# EUROPEAN PATENT APPLICATION

(11) **EP 4 111 983 A1**
(43) Date of publication of application: **04.01.2023**
(21) Application number: 22182456.8
(22) Date of filing: 01.07.2022
(51) Int. Cl.: A61B 8/08, A61B 8/12, A61B 8/00, A61B 5/107

(54) **ULTRASOUND SLICE ENHANCEMENT**

(30) Priority: 02.07.2021 US 202117366267
(71) Applicant: Biosense Webster (Israel) Ltd, Yokneam, 2066717 (IL)
(72) Inventor: WEKSELMAN, Guy, 2066717 Yokneam (IL); SZTEJNBERG, Dan, 2066717 Yokneam (IL); PALTI, Yair, 2066717 Yokneam (IL); GREENBAUM, Lior, 2066717 Yokneam (IL)
(74) Representative: Carpmaels & Ransford LLP

(57) **Abstract**

In one embodiment a system includes a ultrasound probe to capture 2D ultrasonic images of a body part of a living subject, a process to generate a 3D anatomical map of the body part, the 3D anatomical map and the 2D ultrasonic images being registered with a 3D coordinate space, add a 3D indication of an anatomical structure to the 3D anatomical map, render to a display the 3D anatomical map including the 3D indication of the anatomical structure, and render to the display a given one of the 2D ultrasonic images with a 2D indication of the anatomical structure on the given 2D ultrasonic image responsively to the 3D indication of the anatomical structure.

## Description

### FIELD OF THE INVENTION

The present invention relates to medical systems, and in particular, but not exclusively to, mapping.

### BACKGROUND

A variety of devices and methods for intracardiac ultrasonic imaging are known in the art. For example, Biosense Webster Inc. (Irvine, California) offers the CartoSound^{™} system and SoundStar^{™} catheter for producing 3D ultrasound images in real time. The SoundStar^{™} catheter, which is inserted through the vascular system into the heart, contains a position sensor and a phased array of ultrasound transducers. The CartoSound^{™} system processes the signals from the position sensor and the ultrasound transducers to generate 3D images of the heart chambers.

Several methods are known in the art for non-contact reconstruction of the endocardial surface using intracardial ultrasonic imaging. For example, PCT International Publication WO 00/19908 describes a steerable transducer array for intracardial ultrasonic imaging. The array forms an ultrasonic beam, which is steered in a desired direction by an active aperture.

U.S. Patent No. 6,004,269 describes an acoustic imaging system based on an ultrasound device that is incorporated into a catheter. The ultrasound device directs ultrasonic signals toward an internal structure in the heart to create an ultrasonic image.

As another example, PCT International Publication WO 99/55233 describes a method for delineating a 3-D surface of a patient's heart. A 3-D mesh model is developed using training data, to serve as an archetypal shape for a population of patient hearts. Multiple ultrasound images of the patient's heart are taken in different image planes. Anatomical locations are manually identified in each of the images. The mesh model is rigidly aligned with the images in respect to the predefined anatomical locations.

This sort of manual assistance in delineating contours is common in methods for 3D reconstruction based on ultrasound images. For example, U.S. Patent Application Publication No. 2006/0241445 describes a method for modeling of an anatomical structure, in which a plurality of ultrasonic images of the anatomical structure are acquired using an ultrasonic sensor, at a respective plurality of spatial positions of the ultrasonic sensor. Location and orientation coordinates of the ultrasonic sensor are measured at each of the plurality of spatial positions. Contours-of-interest that refer to features of the anatomical structure are marked in one or more of the ultrasonic images. A three-dimensional (3D) model of the anatomical structure is constructed, based on the contours-of-interest and on the measured location and orientation coordinates.

### SUMMARY

There is provided in accordance with an embodiment of the present invention, a medical system, including an ultrasound probe, which is configured to capture a plurality of two-dimensional (2D) ultrasonic images of a body part of a living subject, a display, and a processor configured to generate a three-dimensional (3D) anatomical map of the body part, the 3D anatomical map and the 2D ultrasonic images being registered with a 3D coordinate space, add a 3D indication of an anatomical structure of the body part to the 3D anatomical map, render to the display the 3D anatomical map including the 3D indication of the anatomical structure, and render to the display a given one of the 2D ultrasonic images with a 2D indication of the anatomical structure on the given 2D ultrasonic image responsively to the 3D indication of the anatomical structure.

Further in accordance with an embodiment of the present invention the processor is configured to render to the display the 2D indication of the anatomical structure on the given 2D ultrasonic image, responsively to an intersection, in the 3D coordinate space, of the given 2D ultrasonic image and the 3D indication of the anatomical structure of the 3D anatomical map.

Still further in accordance with an embodiment of the present invention the processor is configured to generate the 3D anatomical map of the body part responsively to the 2D ultrasonic images.

Additionally, in accordance with an embodiment of the present invention the ultrasound probe includes a position sensor configured to provide signals indicative of respective positions of the distal end of the ultrasound probe, the processor being configured to compute the respective positions of the distal end of the ultrasound probe at respective capture times of the 2D ultrasonic images responsively to the provided signals.

Moreover, in accordance with an embodiment of the present invention the processor is configured to generate the 3D anatomical map of the body part responsively to the 2D ultrasonic images and the computed respective positions of the distal end.

Further in accordance with an embodiment of the present invention the position sensor includes a magnetic position sensor to provide the signals indicative of the position of the distal end of the ultrasound probe.

Still further in accordance with an embodiment of the present invention 2D indication of the anatomical structure includes a border of the anatomical structure.

Additionally, in accordance with an embodiment of the present invention 2D indication of the anatomical structure includes a textual identification of the anatomical structure.

There is also provided in accordance with another embodiment of the present invention, a medical method, including capturing a plurality of two-dimensional (2D) ultrasonic images of a body part of a living subject, generating a three-dimensional (3D) anatomical map of the body part, the 3D anatomical map and the 2D ultrasonic images being registered with a 3D coordinate space, adding a 3D indication of an anatomical structure of the body part to the 3D anatomical map, rendering to a display the 3D anatomical map including the 3D indication of the anatomical structure, and rendering to the display a given one of the 2D ultrasonic images with a 2D indication of the anatomical structure on the given 2D ultrasonic image responsively to the 3D indication of the anatomical structure.

Moreover, in accordance with an embodiment of the present invention the rendering includes rendering to the display the 2D indication of the anatomical structure on the given 2D ultrasonic image, responsively to an intersection, in the 3D coordinate space, of the given 2D ultrasonic image and the 3D indication of the anatomical structure of the 3D anatomical map.

Further in accordance with an embodiment of the present invention the generating the 3D anatomical map includes generating the 3D anatomical map of the body part responsively to the 2D ultrasonic images.

Still further in accordance with an embodiment of the present invention, the method includes providing signals indicative of respective positions of the distal end of the ultrasound probe, and computing respective positions of the distal end of the ultrasound probe at respective capture times of the 2D ultrasonic images responsively to the provided signals.

Additionally, in accordance with an embodiment of the present invention the generating the 3D anatomical map includes generating the 3D anatomical map of the body part responsively to the 2D ultrasonic images and the computed respective positions of the distal end.

Moreover, in accordance with an embodiment of the present invention the providing the signals is performed by a magnetic position sensor of the ultrasound probe.

Further in accordance with an embodiment of the present invention 2D indication of the anatomical structure includes a border of the anatomical structure.

Still further in accordance with an embodiment of the present invention 2D indication of the anatomical structure includes a textual identification of the anatomical structure.

There is also provided in accordance with still another embodiment of the present invention, a software product, including a non-transient computer-readable medium in which program instructions are stored, which instructions, when read by a central processing unit (CPU), cause the CPU to receive two-dimensional (2D) ultrasonic images of a body part of a living subject from an ultrasound probe, generate a three-dimensional (3D) anatomical map of the body part, the 3D anatomical map and the 2D ultrasonic images being registered with a 3D coordinate space, add a 3D indication of an anatomical structure of the body part to the 3D anatomical map, render to a display the 3D anatomical map including the 3D indication of the anatomical structure, and render to the display a given one of the 2D ultrasonic images with a 2D indication of the anatomical structure on the given 2D ultrasonic image responsively to the 3D indication of the anatomical structure.

### BRIEF DESCRIPTION OF THE DRAWINGS

The present invention will be understood from the following detailed description, taken in conjunction with the drawings in which:
Fig. 1 is a schematic, pictorial illustration of a catheter-based system for ultrasonic imaging, in accordance with an exemplary embodiment of the present invention;
Fig. 2 is a schematic side view of the distal end of a catheter used in the system of Fig. 1;
Fig. 3 is a schematic representation of an ultrasound image captured by a catheter, in accordance with an exemplary embodiment of the present invention;
Fig. 4 is a flow chart that schematically illustrates a method for fast anatomical mapping using ultrasound images, in accordance with an exemplary embodiment of the present invention;
Fig. 5 is a schematic representation of a 3D map of a heart chamber produced in accordance with an exemplary embodiment of the present invention;
Fig. 6 is a flow chart including steps in a method of operation of the system of Fig. 1; and
Fig. 7 is a schematic view of a 3D anatomical map and a 2D ultrasonic image with anatomical structures disposed thereon.

### DESCRIPTION OF EXAMPLE EMBODIMENTS

### OVERVIEW

As previously mentioned, the SoundStar^{™} catheter, which is inserted through the vascular system into the heart, contains a position sensor and a phased array of ultrasound transducers to collect images of the heart. The CartoSound^{™} system processes the signals from the position sensor and the ultrasound transducers to generate 3D images of the heart chambers.

Ultrasound may provide real-time imaging without the disadvantages of fluoroscopy. The SoundStar^{™} catheter also has the advantage of being inserted into one of the cardiac chambers (e.g., right atrium) and can map all the chambers of the heart. During structural heart procedures, left atrial appendage closure procedures and electrophysiological (EP) cases, a physician may use real time ultrasound images to maneuver a catheter in the heart and image heart structures. However, ultrasound images are not straightforward to understand especially for an inexperienced user. Identifying the anatomical structures on an ultrasound image requires experience.

Embodiments of the present invention solve the above problems by annotating 2D ultrasound images with 2D indications of anatomical structures previously added to a 3D anatomical map thereby making the ultrasound images easier to understand. Three-dimensional indications of 3D anatomical structures may be added to a 3D anatomical map either manually by the physician, or automatically using an auto-segmentation method. For example, structures such as right inferior pulmonary vein (RIPV) and right superior pulmonary vein (RSPV) may be indicated on the 3D anatomical map. As the 3D anatomical map is easier to understand than 2D ultrasound images, it is easier to add the indications of the 3D anatomical structures to the 3D anatomical map. For a given 2D ultrasound image, one or more 2D indications of anatomical structures may be added to that 2D ultrasound image based on how that 2D ultrasound image intersects with the 3D anatomical structure(s) in 3D coordinate space in which the ultrasound images were captured. In other words, the 3D anatomical structures are projected onto corresponding 2D ultrasound images as 2D indications of anatomical structures thereby making the ultrasound images easier to understand and useful during procedures. The 2D indications may include borders (e.g., colored borders to match the colors used for the 3D indication of the different anatomical structures shown on the 3D anatomical map) and/or textual labels, e.g., RIPV or RSPV.

### SYSTEM DESCRIPTION

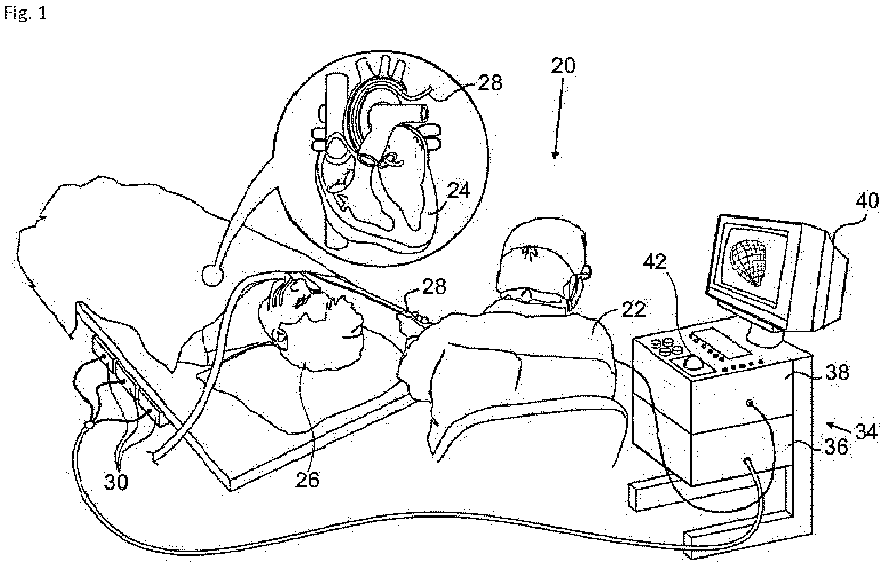

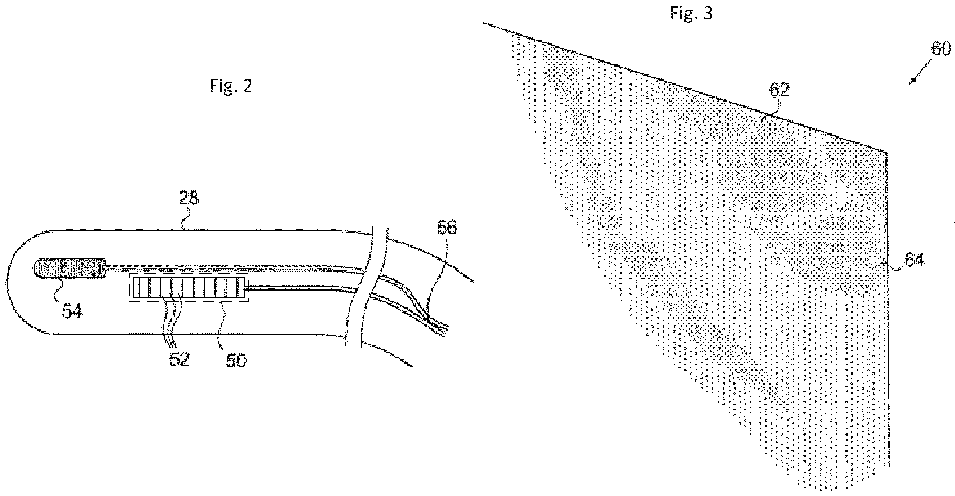

Reference is now made to Figs. 1 and 2, which schematically illustrate a catheter-based ultrasound imaging system 20, in accordance with an embodiment of the present invention. Fig. 1 is a pictorial illustration of the overall system, while Fig. 2 is a side view of the distal end of a probe, such as a catheter 28, that is used in the system. This system and catheter are shown here by way of illustration, to assist in understanding the methods of ultrasound-based 3D mapping that are described further below. These methods, however, are not limited to catheter-based ultrasonic sensing and may similarly be applied, mutatis mutandis, using 2D or 3D ultrasound images acquired by other types of probes, both intra- and extra-corporeal. Furthermore, these methods may be used in mapping of other anatomical cavities, not only in the heart.

As shown in Fig. 1, an operator 22, such as a physician, inserts catheter 28 into the body of a patient 26, so that the distal end of the catheter passes through the vascular system into the patient's heart 24. The catheter is connected at its proximal end to a console 34, which typically comprises a processor 38 with suitable signal processing and user interface circuits. This processor 38 receives and processes signals from catheter 28, as described hereinbelow. Processor 38 may comprise a general-purpose computer processor, which is programmed in software to carry out the functions that are described herein. This software may be downloaded to the processor in electronic form, over a network, for example. Alternatively, or additionally, the software may be stored on tangible computer-readable storage media, such as optical, magnetic, or electronic memory media. Further additionally or alternatively, at least some of the functions of the processor may be carried out by a digital signal processor (DSP) or by dedicated or programmable hardware logic circuits.

Typically, console 34 also enables a user to observe and regulate the functions of catheter 28 and to view and edit images that are formed using the catheter. For these purposes, the console comprises a display 40 and a user interface 42.

As shown in Fig. 2, the distal end of catheter 28 comprises an ultrasound imaging device (or probe) 50, which is used to produce ultrasound images of the inside of the body. Device 50 typically comprises a phased array of transducers 52, which is operated, as is known in the art, so as to capture a two-dimensional (2D) "fan" image in the plane of the scanning ultrasonic beam (referred to herein as the "beam plane" or "image plane"), which contains the longitudinal axis of the catheter 28. The transducers 52 receive ultrasonic waves that are reflected from objects in the beam plane and output signals in response to the reflected waves. Typically, these signals are conveyed by wires 56 running through catheter 28 to console 34, which processes the signals in order to form and display ultrasound images and 3D maps, as described hereinbelow.

The distal end of catheter 28 further comprises a position sensor 54, which generates signals that indicate the position (location and orientation including roll) of the catheter within the body. Based on these position signals, console 34 determines the location and orientation of each fan image captured by imaging device 50. Processor 38 is thus able to determine the coordinates of objects appearing in the fan image, as well as to register and combine multiple 2D images captured at different catheter positions.

In the pictured embodiment, system 20 uses magnetic position sensing to determine position coordinates of the distal end of catheter 28 inside heart 24. To determine the position coordinates, a driver circuit 36 in console 34 drives field generators 30 to generate magnetic fields within the body of patient 26. Typically, field generators 30 comprise coils, which are placed below the patient's torso at known positions external to the body. These coils generate magnetic fields in a predefined working volume that contains heart 24. Sensor 54, which may comprise, for example, a magnetic position sensor including one or more coils within the distal end of catheter 28, generates electrical signals in response to these magnetic fields. Processor 38 processes these signals in order to determine the position (location and orientation) coordinates of the distal end of catheter 28. Console 34 may use the coordinates in driving display 40 to show the location and status of the catheter.

This method of position sensing and processing is implemented in the CARTO^{®} 3 system produced by Biosense Webster Inc. This sort of magnetic position sensing is described in detail, for example, in U.S. Patent No. 6,266,551. Other systems that combine ultrasonic imaging with magnetic position sensing are described in U.S. Patent Nos. 6,690,963, 6,716,166 and 6,773,402.

Alternatively, or additionally, system 20 may comprise an automated mechanism (not shown) for maneuvering and operating catheter 28 within the body of patient 26. In such embodiments, processor 38 generates a control input for controlling the motion of the catheter based on the signals provided by the position sensing system.

Although Fig. 1 shows a particular system configuration, other system configurations may be used in alternative embodiments of the present invention. For example, the methods described hereinbelow may be applied using position transducers of other types, such as impedance-based or ultrasonic position sensors. The term "position transducer" as used herein refers to an element mounted on or in catheter 28 that causes console 34 to receive signals indicative of the coordinates of the element. The position transducer may thus comprise a receiver in the catheter, such as sensor 54, which generates a position signal to the control unit based on energy received by the transducer; or it may comprise a transmitter, emitting energy that is sensed by a receiver external to the probe. Furthermore, the methods described hereinbelow may similarly be applied in mapping and imaging applications using not only catheters, but also probes of other types, both in the heart and in other body organs and regions, as well as ultrasound probes external to the body.

Reference is now made to Fig. 3, which is a schematic representation of an ultrasound image 60 captured by catheter 28, in accordance with an embodiment of the present invention. The image has the form of a 2D fan, with its vertex at imaging device 50. As noted above, console 34 can determine the location of the vertex and the orientation of the fan in 3D space based on the signals received from position sensor 54. Dark areas 62, 64 in the image correspond to areas, such as the heart chambers, that are filled with blood and therefore have low reflectance. Brighter areas generally represent tissue, such as the internal and external heart walls.

As noted earlier, operator 22 may manipulate catheter 28 inside heart 24 to capture images from different locations and at different orientations. The reflections that make up the images may originate not only from the heart chamber in which the distal end of the catheter 28 is located, but also from other heart chambers and anatomical structures. Thus, for example, catheter 28 may be inserted into the right atrium (which is relatively easy to access via the vena cava) and may capture images from the right atrium of the left atrium and possibly the ventricles.

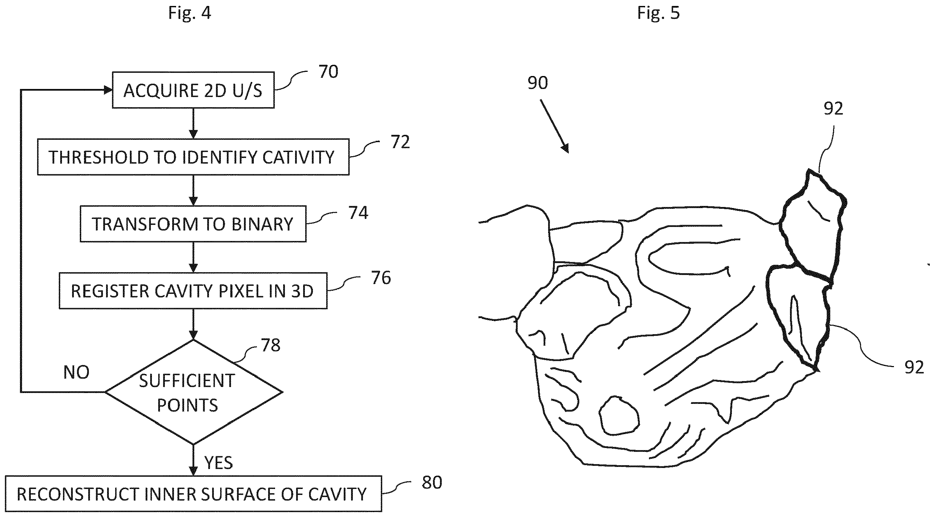

Fig. 4 is a flow chart that schematically illustrates a method for fast anatomical mapping using ultrasound images, in accordance with an embodiment of the present invention. At each iteration of the method, sensor 50 acquires a 2D ultrasound fan image, having the general form of image 60 (Fig. 3), at an image acquisition step (block 70). Image acquisition may be gated to a certain annotation point in the heart cycle (such as systole or diastole), using an electrocardiogram (ECG) monitor for synchronization, for example, or the images may alternatively be acquired continuously, without gating. Processor 38 identifies the inside of the heart chamber of interest (the blood pool area) in each 2D image acquired by the ultrasound catheter, at a cavity identification step (block 72). These "dark," low-reflectance areas may be identified, for example, by applying a threshold to the gray scale levels of the ultrasound image. The threshold may be set automatically or manually. Any suitable method known in the art may be used to choose the threshold automatically, such as the Otsu method, in which the threshold is chosen based on clustering of the pixel gray-scale values.

Alternatively, other ultrasound imaging modes may be used in image capture at the step of block 70, and the method used to identify the cavity at the step of block 72 may be adapted accordingly. For example, the 2D images may be acquired using Doppler imaging techniques, such as Color Doppler, Power Doppler or Tissue Imaging Doppler, as are known in the art. Such techniques use image color (commonly referred to as pseudo-color) to represent flow. In Color Doppler, areas of blood flow are colored in the image, while areas of tissue are not. In this case, pixels having a color value above a certain threshold may be identified as belonging to the blood pool area at the step of block 72. On the other hand, in Tissue Imaging Doppler, areas of tissue are colored while blood is not, so that pixels having a color value below a certain threshold will be identified as belonging to the blood pool area.

Whichever imaging modality is used, processor 38 applies the threshold to transform the 2D grayscale or color image into a binary image, at a binarization step (block 74). In the binary image, pixels with the value '0' are classified as belonging to areas of blood, while pixels with the value '1' belong to tissue. Other image processing operations may be applied in order to improve the precision of separation between blood and tissue separation. For example, morphological erosion and dilation may be applied in succession to remove small dark areas, which may have been misidentified as blood pool areas, within tissue regions.

Processor 38 finds the vertex location and the orientation of the captured 2D image, at an image registration step (block 76). As noted above, the processor may compute the location and orientation coordinates on the basis of the signals output by position sensor 54. Given the vertex position and image orientation, the processor 38 can calculate the 3D coordinates of every pixel in the binarized image in the fixed 3D reference frame (defining a 3D coordinate space) of field generators 30, and thus registers the 2D image pixels in the 3D volume.

After capturing each 2D image, the operator moves the catheter tip in the heart, and the above 2D image capture and processing steps are repeated until the processor has collected a sufficient number of binary pixel values within the volume of interest, at a capture completion step (block 78).

Processor 38 collects the pixels with value '0' (blood) and finds the outer surface bounding these pixels, at a surface reconstruction step (block 80). The outer surface bounding the blood pool in a heart chamber is the inner surface of the heart wall surrounding that chamber. Thus, by finding the bounding outer surface of the blood pool the processor has, in effect, constructed a 3D anatomical map of the chamber in question. Optionally, the processor may continue acquiring 2D images while reconstructing the 3D surface and may thus refine the map progressively.

Using the blood pool in the 3D domain as the basis for surface reconstruction has the advantage of offering a fast, efficient way to overcome the problem of image segmentation. Various algorithms may be used to reconstruct the outer surface of a volume based on a collection of interior points of this sort. For example, processor 38 may apply the ball-pivoting algorithm that is described in U.S. Patent No. 6,968,299. This algorithm computes a triangle mesh interpolating a given point cloud by "rolling" a ball of a certain radius over the point cloud. The vertices of the triangles that are found in this way define the outer surface of the cloud.

To reduce the computational load, not all the '0' pixels are necessarily used in building the model, and processor 38 may apply different resolution levels in different areas. The above-mentioned ball-pivoting algorithm may be adapted for this sort of variable-resolution mapping by using different ball radii in different regions of the map. High resolution is typically needed only in the blood-tissue interface area (along the edge of the blood pool). This area can be identified automatically or manually in the binary images. The processor 38 then performs high-resolution reconstruction only near the tissue boundary, and uses low resolution elsewhere. In other words, the processor 38 uses fewer pixels from the 2D images in areas far from the tissue, and more pixels in the area of the tissue interface (typically within a thickness on the order of 1 mm).

After processor 38 has reconstructed and displayed the 3D anatomical map in the manner described above, a user may apply image editing tools, via user interface 42, for example, to cut and/or morph the model in order to correct artifacts and remove features that are not of interest. In some embodiments, the user may add indications of anatomical structures such as the right inferior pulmonary vein (RIPV) and the right superior pulmonary vein (RSPV) to the 3D anatomical map.

Fig. 5 is a schematic representation of a 3D anatomical map 90 of a heart chamber, of a type that may be produced by the above method in accordance with an embodiment of the present invention. The map in this case shows the right atrium of the heart, including parts of the pulmonary veins. This sort of map may be used for various purposes, such as serving as a starting point for an electro-anatomical map (of the type produced by the above-mentioned CARTO 3 system) or for segmentation of a full 3D ultrasonic or tomographic image. The map 90 may also include indications 92 of anatomical structures such as the RIPV and the RSPV added by the user or by an auto-segmentation method.

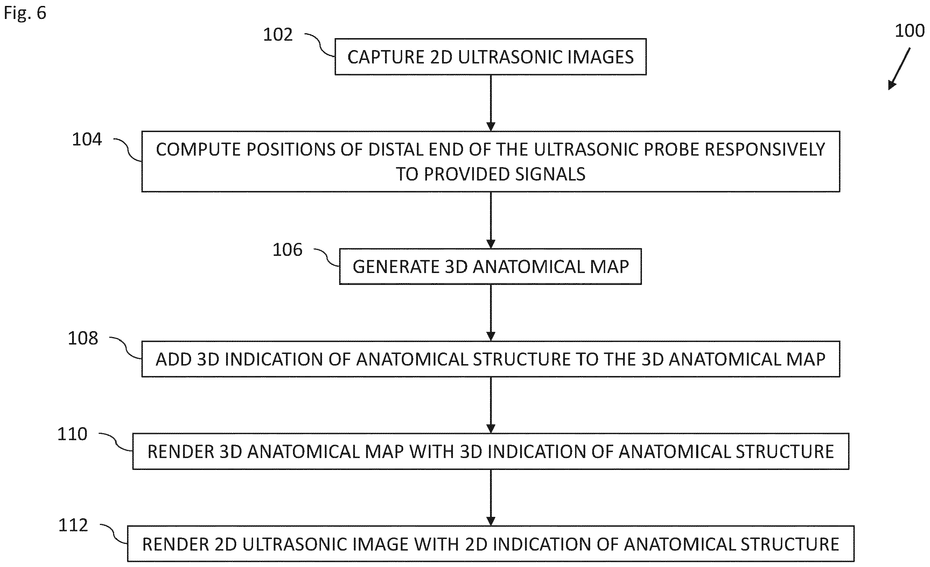

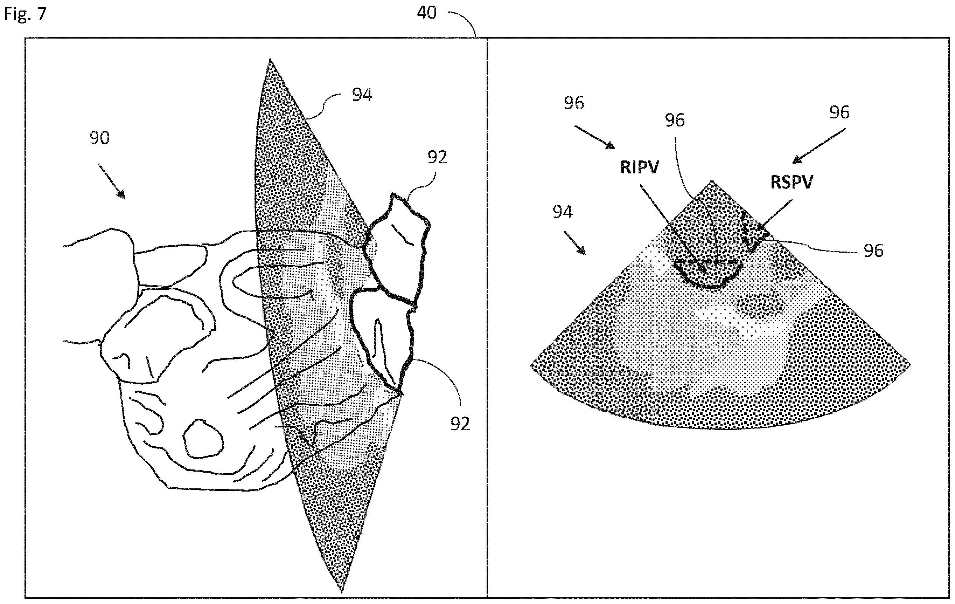

Reference is now made to Figs. 6 and 7. Fig. 6 is a flow chart 100 including steps in a method of operation of the system 20 of Fig. 1. Fig. 7 is a schematic view of the 3D anatomical map 90 and a 2D ultrasonic image 94 with indications 92, 96 of anatomical structures disposed thereon, respectively. Prior to describing the steps of flow chart 100 in more detail, the display shown in Fig. 7 is now described.

The right-hand side of the display 40 shown in Fig. 7 shows a selected 2D ultrasonic image 94. The 2D ultrasonic image 94 may be selected from a gallery of captured 2D ultrasonic images or from a video including a plurality of 2D ultrasonic images taken over time as the catheter 28 is moved in the body part. The 2D ultrasonic image 94 may also be selected by selecting a plane or 2D slice from the 3D anatomical map 90. The closest 2D ultrasonic image to the plane or 2D slice is then selected and shown in the right-hand side of the display 40.

The left-hand side of the display 40 shows the 3D anatomical map 90 with the 3D indications 92 of the anatomical structures thereon. The 3D indications 92 may be added by the user and/or using an auto-segmentation method, which identifies anatomical structures automatically from the 3D anatomical map 90 or from physiological data such as intracardiac electrograms, pressure readings and/or temperature readings. In the example of Fig. 7, the top anatomical structure is the RSPV and the anatomical structure below that is the RIPV. The 2D ultrasonic image 94 is also shown intersecting the 3D anatomical map 90. The 2D ultrasonic image 94 is shown as being partially transparent so that the orientation of the 2D ultrasonic image 94 with respect to the 3D anatomical map 90 can clearly be seen. In some embodiments, the 2D ultrasonic image 94 is shown as being opaque over the 3D anatomical map 90. The 2D ultrasonic image 94 is positioned with respect to the 3D anatomical map 90 according to the respective positions of the 3D anatomical map 90 and the 2D ultrasonic image 94 in the 3D coordinate space according to the positions provided by the position sensor 54 and the associated position tracking system described above with reference to Figs. 1-4.

The right-hand side of the display 40 also shows 2D indications 96 of the anatomical structures identified on the 3D anatomical map 90. The 2D indications 96 are shown on the 2D ultrasonic image 94 according to where the 3D indications 92 of the anatomical structures intersect the 2D ultrasonic image 94 in the 3D coordinate space. The indications 96 also include textual indications (e.g., RIPV and RSPV) to clearly indicate the respecting anatomical structures intersecting the 2D ultrasonic image 94.

The steps of flow chart 100 are now described in more detail.

The ultrasound imaging device 50 is configured to capture (block 102) a plurality of two-dimensional (2D) ultrasonic images of a body part (e.g., one or more chambers of the heart 24 of Fig. 1) of a living subject (e.g., the patient 26 of Fig. 1).

The position sensor 54 of the ultrasound imaging device 50 (e.g., ultrasound probe) is configured to provide signals indicative of respective positions of the distal end of the ultrasound imaging device 50 as described above with reference to Fig. 1 and 2. In some embodiments, the position sensor 54 comprises a magnetic position sensor to provide the signals indicative of the position of the distal end of the ultrasound imaging device 50. The processor 38 (Fig. 1) is configured to compute (block 104) respective positions of the distal end of the ultrasound imaging device 50 at respective capture times of the 2D ultrasonic images responsively to the provided signals. In other words, each time one of the 2D ultrasonic images is captured, the signal(s) provided by the position sensor 54 may be processed to compute a position of that captured 2D ultrasonic image so that positions of the pixels of that 2D ultrasonic image may be computed in a 3D coordinate space, as described in more detail with reference to Fig. 4. In some embodiments, the position of the distal end of the ultrasound imaging device 50 may be computed using any suitable position tracking method.

The processor 38 (Fig. 1) is configured to generate (block 106) the 3D anatomical map 90 of the body part. The 2D ultrasonic images and the 3D anatomical map 90 are registered with the 3D coordinate space. In some embodiments, the processor 38 is configured to generate the 3D anatomical map 90 of the body part responsively to the captured 2D ultrasonic images and the computed respective positions of the distal end. For example, pixels of the 2D ultrasonic images and the corresponding computed positions of the pixels provide the boundaries of the inner surface of the body part as described above in detail with reference to Fig. 4. For example, the processor 38 finds pixels with value '0' (blood) and the outer surface bounding these pixels, at a surface reconstruction step (block 80 of Fig. 4). The outer surface bounding the blood pool in a heart chamber is the inner surface of the heart wall surrounding that chamber. Thus, by finding the bounding outer surface of the blood pool the processor has, in effect, constructed the 3D anatomical map 90 of the chamber in question.

The 3D anatomical map 90 may be generated using any suitable method, for example, manually annotating the outer surface on each of the captured 2D ultrasonic images and then generate the 3D anatomical map 90 from the manual annotations, or using a catheter with one or more electrodes to inserted into the body part to identify positions of the outer surface.

The processor 38 (Fig. 1) is configured to add (block 108) the 3D indication(s) 92 of the corresponding anatomical structure(s) of the body part to the 3D anatomical map 90. The indications 92 may be added by the operator 22 drawing the boundaries of the anatomical structures on the 3D anatomical map 90 or using an auto-segmentation method, which identifies anatomical structures automatically from the 3D anatomical map 90 or from other physiological data including intracardiac electrograms, pressure and/or temperature readings and adds the 3D indications 92 of the identified anatomical structures to the 3D anatomical map 90. The indications 92 may include a 3D border around the anatomical structures, and/or a 3D shading (e.g., color or grayscale) or pattern fill to identify the anatomical structures. The processor 38 is configured to render (block 110) to the display 40 the 3D anatomical map 90 including the 3D indication(s) 92 of the anatomical structure(s).

The processor 38 (Fig. 1) is configured to render (block 112) to the display 40 a given (e.g., selected) one of the 2D ultrasonic images (e.g., the 2D ultrasonic image 94) with 2D indication(s) 96 of the anatomical structure(s) on the given 2D ultrasonic image. The 2D indication(s) 96 of the anatomical structure(s) may include a border (or borders) of the anatomical structure(s) and/or a textual identification(s) (e.g., "RIPV" and "RSPV") of the anatomical structure(s). In some embodiments, the processor 38 is configured to render to the display 40 the 2D indication(s) 96 of the anatomical structure(s) on the given 2D ultrasonic image, responsively to an intersection, in the 3D coordinate space, of the given 2D ultrasonic image (e.g., the 2D ultrasonic image 94) and the 3D indication(s) 92 of the anatomical structure(s) of the 3D anatomical map 90. In other words, the 3D indication(s) 92 may be projected on to the 2D ultrasonic image 94 as 2D indication(s) 96 according to the intersection of the 3D indication(s) 92 with the 2D ultrasonic image 94 responsively to the position of the 2D ultrasonic image 94 and the 3D indications 92 in the 3D coordinate space.

It should be noted that the 2D indications 96 are not added by the operator 22 or using an auto-segmentation method to respective 2D ultrasonic images. The 3D indications 92 of the anatomical structures are first added by the operator 22 (and/or by an auto-segmentation method) to the 3D anatomical map 90. The 3D indications 92 are then projected from the 3D anatomical map 90 to the respective 2D ultrasonic images as 2D indications 96 according to the respective positions of the 2D ultrasound images in the 3D coordinate space and the positions of the 3D indications 92 in the 3D coordinate space.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 72% to 108%.

Various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination.

The embodiments described above are cited by way of example, and the present invention is not limited by what has been particularly shown and described hereinabove. Rather the scope of the invention includes both combinations and sub-combinations of the various features described hereinabove, as well as variations and modifications thereof which would occur to persons skilled in the art upon reading the foregoing description and which are not disclosed in the prior art.

## Claims

1. A medical system, comprising:
an ultrasound probe, which is configured to capture a plurality of two-dimensional (2D) ultrasonic images of a body part of a living subject;
a display; and
a processor configured to:
generate a three-dimensional (3D) anatomical map of the body part, the 3D anatomical map and the 2D ultrasonic images being registered with a 3D coordinate space;
add a 3D indication of an anatomical structure of the body part to the 3D anatomical map;
render to the display the 3D anatomical map including the 3D indication of the anatomical structure; and
render to the display a given one of the 2D ultrasonic images with a 2D indication of the anatomical structure on the given 2D ultrasonic image responsively to the 3D indication of the anatomical structure.

2. The system according to claim 1, wherein the processor is configured to render to the display the 2D indication of the anatomical structure on the given 2D ultrasonic image, responsively to an intersection, in the 3D coordinate space, of the given 2D ultrasonic image and the 3D indication of the anatomical structure of the 3D anatomical map.

3. The system according to claim 1, wherein the processor is configured to generate the 3D anatomical map of the body part responsively to the 2D ultrasonic images.

4. The system according to claim 1, wherein the ultrasound probe includes a position sensor configured to provide signals indicative of respective positions of the distal end of the ultrasound probe, the processor being configured to compute the respective positions of the distal end of the ultrasound probe at respective capture times of the 2D ultrasonic images responsively to the provided signals.

5. The system according to claim 4, wherein the processor is configured to generate the 3D anatomical map of the body part responsively to the 2D ultrasonic images and the computed respective positions of the distal end.

6. The system according to claim 4, wherein the position sensor comprises a magnetic position sensor to provide the signals indicative of the position of the distal end of the ultrasound probe.

7. A medical method, comprising:
capturing a plurality of two-dimensional (2D) ultrasonic images of a body part of a living subject;
generating a three-dimensional (3D) anatomical map of the body part, the 3D anatomical map and the 2D ultrasonic images being registered with a 3D coordinate space;
adding a 3D indication of an anatomical structure of the body part to the 3D anatomical map;
rendering to a display the 3D anatomical map including the 3D indication of the anatomical structure; and
rendering to the display a given one of the 2D ultrasonic images with a 2D indication of the anatomical structure on the given 2D ultrasonic image responsively to the 3D indication of the anatomical structure.

8. The method according to claim 7, wherein the rendering includes rendering to the display the 2D indication of the anatomical structure on the given 2D ultrasonic image, responsively to an intersection, in the 3D coordinate space, of the given 2D ultrasonic image and the 3D indication of the anatomical structure of the 3D anatomical map.

9. The method according to claim 7, wherein the generating the 3D anatomical map includes generating the 3D anatomical map of the body part responsively to the 2D ultrasonic images.

10. The method according to claim 7, further comprising:
providing signals indicative of respective positions of the distal end of the ultrasound probe; and
computing respective positions of the distal end of the ultrasound probe at respective capture times of the 2D ultrasonic images responsively to the provided signals.

11. The method according to claim 10, wherein the generating the 3D anatomical map includes generating the 3D anatomical map of the body part responsively to the 2D ultrasonic images and the computed respective positions of the distal end.

12. The method according to claim 10, wherein the providing the signals is performed by a magnetic position sensor of the ultrasound probe.

13. The system according to claim 1 or the method according to claim 7, wherein 2D indication of the anatomical structure includes a border of the anatomical structure.

14. The system according to claim 1 or the method according to claim 7, wherein 2D indication of the anatomical structure includes a textual identification of the anatomical structure.

15. A software product, comprising a non-transient computer-readable medium in which program instructions are stored, which instructions, when read by a central processing unit (CPU), cause the CPU to:
receive two-dimensional (2D) ultrasonic images of a body part of a living subject from an ultrasound probe;
generate a three-dimensional (3D) anatomical map of the body part, the 3D anatomical map and the 2D ultrasonic images being registered with a 3D coordinate space;
add a 3D indication of an anatomical structure of the body part to the 3D anatomical map;
render to a display the 3D anatomical map including the 3D indication of the anatomical structure; and
render to the display a given one of the 2D ultrasonic images with a 2D indication of the anatomical structure on the given 2D ultrasonic image responsively to the 3D indication of the anatomical structure.
